# DEMANDE DE BREVET EUROPEEN

(11) **EP 1 228 765 A1**
(43) Date de publication de la demande: **07.08.2002**
(21) Numéro de dépôt: 01102218.3
(22) Date de dépôt: 31.01.2001
(51) Int. Cl.: A61K 35/84, A61P 9/10

(54) **Agent hypocholestérolémiant**

(71) Demandeur: SOCIETE DES PRODUITS NESTLE S.A., 1800 Vevey (CH)
(72) Inventeur: Hajjaj, Hassan, 1073 Savigny (CH); Mace, Catherine, 1095 Lutry (CH); Niederberger, Peter, 1066 Epalinges (CH); Fay, Laurent, Bernard, 74500 Evian (FR)
(74) Mandataire: Wavre, Claude-Alain

(57) **Abrégé**

La présente invention concerne un agent hypocholestérolémiant riche en dérivés naturels de lanostérol oxygénés issus de champignons comestibles. Un tel agent peut être additionné à des produits alimentaires.

Procédé de fabrication d'un tel agent.

## Description

La présente invention concerne un agent hypocholestérolémiant issu de champignons comestibles.

Actuellement les considérations nutritionnelles des consommateurs poussent les industriels de l'alimentation à proposer des aliments présentant des fonctionnalités nutritionnelles améliorées.

Il est reconnu qu'un taux élevé de cholestérol sérique est un facteur non négligeable de risque athéroscléreux. Ainsi, la diminution de ce taux de cholestérol sérique est un moyen de lutte contre le risque de maladies cardio-vasculaires. Sachant que la majeure partie du cholestérol circulant est issue d'une synthèse *de novo,* l'inhibition de la biosynthèse de cholestérol endogène apparaît comme une voie intéressante de traitement, ou de prévention de l'hypercholestérolémie.

La biosynthèse du cholestérol comprend trois étapes majeures. La première, la condensation, permet de passer de trois acétyl-CoA au mévalonate. Cette condensation implique la participation, entre autres, de la 3-Hydroxy-3-methylglutaryl-CoA-reductase (HMGCoA-reductase). Le mévalonate ainsi formé est ensuite polymérisé en squalène via des intermédiaires polyisoprène. Enfin, le squalène mène au cholestérol par cyclisation/déméthylation via la formation d'intermédiaires réactionnels, entre autres le lanostérol et ses dérivés.

Différents composés sont utilisés pour inhiber la synthèse du cholestérol. Les statines, en particulier, agissent très en amont, par inhibition de HMGCoA-reductase. La lovastatine, isolée et purifiée à partir de divers champignons et/ou moisissures a été mise à profit comme agent hypocholestérolémiant sous forme de médicament ou de complément nutritionnel. Ainsi DE 4402591 décrit la production et l'isolement de lovastatine et d'acide mevinolinique de *Pleurotus ostreatus,* ainsi que l'utilisation de ces composés pour inhiber la biosynthèse du cholestérol. L'utilisation de lovastatine nécessite cependant un isolement ainsi qu'une purification avant son emploi. D'autre part, les composés de la classe des statines, inhibiteurs de HMGCoA-reductase induisent des effets secondaires indésirables. En effet l'HMGCoA-reductase est une enzyme qui agit très en amont dans la voie de la chaîne de biosynthèse du cholestérol (transformation de l'HMGCoA en mévalonate), dès lors son inhibition tend à modifier la synthèse d'autres composés dérivés du mévalonate. Ainsi, en supprimant la production de mévalonate, on observe une diminution de la synthèse de divers stéroïdes, hormones, de co-enzyme Q10, d'isopentenyl ARNt, de dérivés isoprenoïdes ou de dolichol par exemple. La déplétion de tels métabolites est responsable, entre autres, de troubles hépatiques et musculaires via une augmentation du taux de transaminases hépatiques, par exemple (Hiyoshi et al, J. of Lipid Research, 2000, (41) 1136-1141).

Dans cette optique, on a cherché des composés capables d'inhiber les étapes terminales de la chaîne de synthèse du cholestérol pour éviter les problèmes inhérents aux inhibiteurs de HMGCoA-reductase. Divers composés de synthèse, inhibiteurs des étapes terminales de déméthylation du lanostérol en 14 déméthyl-lanostérol, existent. Ces composés sont pour la plupart des dérivés oxygénés de stérols.

Cependant, tous ces inhibiteurs sont des purs produits de synthèse chimique, tels ceux décrits dans WO9113903. Ainsi, outre le fait que leur fabrication passe par une synthèse chimique longue, complexe et onéreuse, ces composés entrent dans la classe des médicaments, ce qui implique des études cliniques pour homologation, enregistrement et autorisation.

Komoda et al (Chem. Pharm. Bull., 1989 37(2) 531-533) décrivent des dérivés de lanostérol oxygénés obtenus par modification chimique d'acide Ganodérique isolé de Ganoderma lucidum. Il est indiqué dans ce document que seul les dérivés modifiés par voie chimique présentent une action hypocholestérolémiante. L'acide ganodérique B ainsi que son dérivé ester, servant à l'obtention des divers autres dérivés, ne présentent pas d'activité inhibitrice sur la synthèse du cholestérol.

Différents champignons sont décrits pour leurs propriétés hypocholestérolémiantes. Ainsi KR 9303886 décrit la préparation d'aliments hypocholestérolémiants à partir de champignons médicinaux par séchage, pulvérisation, extraction à l'eau bouillante et concentration. Il apparaît que de tels extraits agissent au niveau de HMGCoA-réductase (Bobek et al, Casopis Lebaru Ceskych, 1997 136(6) 186-190 ; Bobek et al, Experientia, 1995 51(6) 589-591). DE 4402591, mentionné plus haut, suggère aussi que certains champignons comestibles sont riches en inhibiteurs de HMGCoA-réductase.

Le but de la présente invention est de fournir un agent hypocholestérolémiant, non issu de synthèse chimique et utilisable dans un produit alimentaire, agissant sur les étapes terminales de la chaîne de biosynthèse du cholestérol.

A cette fin, l'agent hypocholestérolémiant selon la présente invention comprend des dérivés naturels de lanostérol oxygénés issus de champignons comestibles.

Par « agent hypocholestérolémiant », on entend un agent qui inhibe la synthèse du cholestérol et plus particulièrement en agissant sur les étapes terminales de la chaîne de biosynthèse du cholestérol, et ceci grâce à son contenu en dérivés naturels de lanostérol oxygénés.

Par « dérivés naturels » on entend des composés qui n'ont subi aucune modification chimique par quelque moyen que ce soit. Il s'agit des dérivés de lanostérol oxygénés obtenus directement à partir des champignons comestibles.

De même, par « champignons comestibles », on entend, des champignons avec une longue tradition de consommation alimentaire. On entend ainsi tant des champignons ni toxiques ni vénéneux, qui se distinguent ou non par leurs qualités gustatives et/ou aromatiques. Il peut s'agir par exemple des champignons choisis dans le groupe comprenant les ordres de classification suivants : Agaricalles, Aphyllophorales et Stereales. De manière préférentielle, il peut s'agir de champignons choisis dans le groupe comprenant : *Pleurotus eryngii*, *Pleurotus eous*, *Ganoderma lucidum, Grifola frondosa, Pleurotus ostreatus, Agrocybe aegerita, Pholiota nameko, Pleurotus citrinopileatus et Flamulina velutipes.*

L'invention concerne aussi l'utilisation dans un produit alimentaire d'un agent hypocholestérolémiant comprenant des dérivés naturels de lanostérol oxygénés issus de champignons comestibles.

Enfin, le procédé de préparation de l'agent hypocholestérolémiant selon la présente invention comprend les étapes suivantes :
- macération d'un champignon comestible dans un premier solvant,
- séparation des phases solides et liquides,
- évaporation de la phase liquide jusqu'à obtention d'un extrait sec,
- reprise de l'extrait sec avec de l'eau,
- extraction de la phase aqueuse à l'aide d'un second solvant de polarité inférieure au premier solvant et non-miscible avec l'eau et
- séparation des phases aqueuse et organique.

L'extrait organique ainsi obtenu après élimination de la phase aqueuse représente l'agent hypocholestérolémiant riche en dérivés de lanostérol naturels oxygénés et est utilisé pour les évaluations in vitro, les fractionnements et purifications.

L'étape de macération peut être précédée d'une étape de broyage et s'effectuer avec du champignon frais ou séché. Cette macération peut s'effectuer à température ambiante ou à une température de réfrigération. Selon la température de macération choisie, cette dernière s'effectuera sur une durée plus ou moins longue. L'étape de macération peut ainsi s'étaler sur une durée pouvant être comprise entre 6 et 72 heures. Le premier solvant utilisé pour cette macération peut être du méthanol, de l'éthanol ou du chloroforme, par exemple, utilisés seuls ou en mélange.

La séparation des phases solide et liquide peut être réalisée par filtration sur papier ou sur gaze, par centrifugation ou par décantation, par exemple.

Après évaporation de la phase liquide, de préférence à une température proche de la température ambiante, l'extrait sec obtenu peut être repris avec une quantité d'eau comprise entre 5 et 100 % (v/v) par rapport à la quantité de premier solvant utilisé pour la macération. De manière à faciliter l'extraction avec le second solvant, on peut ajuster le pH de cette solution aqueuse ainsi obtenue à une valeur de pH acide, c'est à dire à une valeur comprise entre 2 et 4, par exemple, avant l'extraction à l'aide du second solvant. Cette acidification peut être réalisée à l'aide d'acide chlorhydrique, par exemple.

La solution aqueuse ainsi obtenue peut être soumise à une extraction à l'aide d'un second solvant non miscible avec l'eau et de polarité inférieure au premier tel que de l'acétate d'éthyle, de l'isopropanol ou du chloroforme par exemple, volume à volume. Le deuxième solvant sera choisi selon ce doublé critère de polarité inférieure au premier solvant et de non-miscibilité avec l'eau. Dans le cas particulier de l'utilisation possible du chloroforme comme premier solvant, par exemple, celui-ci ne constituera pas le deuxième solvant et ce second solvant sera choisi de polarité inférieure au chloroforme et non miscible avec l'eau. Une telle extraction peut être réalisée plusieurs fois consécutivement, par exemple. La phase organique peut être récupérée par décantation, par exemple, et évaporée, de préférence sous vide à une température de l'ordre de 25 à 35 °C. Avant cette étape d'évaporation, la phase organique peut éventuellement être séchée, par exemple à l'aide de Na₂SO₄ anhydre.

Ainsi, une fois la phase organique complètement évaporée on obtient un extrait sous forme pulvérulente riche en dérivés de lanostérol naturels oxygénés. Un tel extrait peut être additionné dans un produit alimentaire, par exemple.

Dans le présent contexte, l'effet hypocholestérolémiant du présent produit est estimé de manière qualitative sur le modèle de cellules hépatiques humaines T9A4 en culture *in vitro.* Bien que l'on sache que de tels résultats ne sont pas directement transférables *in vivo* à l'homme, ils n'en fournissent pas moins des indications utiles. L'effet hypocholestérolémiant est estimé par la mesure de la synthèse *de novo* de cholestérol sur des cellules hépatiques humaines en culture en mesurant l'incorporation d'acétate-C¹⁴. Un mode d'évaluation de cette synthèse *de novo* est décrit dans une méthode présentée ci-après.

L'agent obtenu après extraction à partir de champignon peut être purifié, fractionné, analysé et caractérisé par chromatographie liquide et gazeuse, spectroscopie de masse et résonance magnétique nucléaire de manière à identifier les dérivés de lanostérol oxygénés contenus. Les matériels et les méthodes mis en oeuvre pour une telle caractérisation sont décrits en détail dans des méthodes présentées ci-après.

### Analyse in vitro de l'effet hypocholestérolémiant

Les cellules hépatiques humaines T9A4 sont mises en croissance sur le milieu LCM (Biofluids, Rockville, MD) à 37°C sous 3,5% CO2. Les cellules sont ensemencées dans des boites de cultures à 24 puits et incubées jusqu'à confluence avec 1 mM acétate C14 (1 mCi/mol, Amersham) pendant 20 heures en l'absence (contrôle) ou en présence de l'extrait riche en dérivés de lanostérol oxygénés issus de champignons et/ou de fractions purifiées, dissout dans le méthanol.

Une extraction de lipides est réalisée par incubation dans un mélangé hexane:isopropanol (3:2) pendant 30 minutes à température ambiante. L'extrait est ensuite séché sous azote puis redissout dans de l'hexane et soumis à une chromatographie haute performance en couche mince (Merck, Darmstadt, Allemagne) avec comme solvant un mélange hexane :diethyl ether:acide acétique (75:25:1). La neo-synthèse de cholestérol est déterminée en mesurant l'incorporation de l'acétate C¹⁴ au sein du cholestérol à l'aide d'un imageur (Camberra Packard, Zurich, Suisse) et exprimée en % du contrôle.

Les résultats sont présentés dans le tableau 1. ID50 représente la dose inhibant la synthèse de cholestérol de 50%. On peut noter que les différents extraits présentent une activité hypocholestérolémiante notable. De plus, les extraits obtenus avec *Ganoderma lucidum, Pleurotus citrinopileatus* et *Flamulina velutipes* montrent une activité très forte avec un seuil d'inhibition 50% de l'ordre de 1 à moins de 14 µg/ml.

Les extraits bruts actifs de *Ganoderma lucidum* et de *Pleurotus citrinopileatus* ont été purifiés et testés pour leur activités hypocholestérolémiante. Les résultats sont présentés au tableau 2. On peut constater que l'extrait brut obtenu à partir de *Ganoderma lucidum* contient lui-même une grande quantité de composés hypocholestérolémiants dont la dose inhibant 50 % de la biosynthèse du cholestérol est inférieure à 3 µg/ml. L'inhibition observée avec les fractions purifiées à partir de *Pleurotus ostreatus* présentent un ID50 plus élevée mais cependant particulièrement intéressante.

### Caractérisation physico-chimique des composés contenus dans les extraits de champignons.

### Spectrométrie de masse

### HPLC/SM (chromatographie liquide haute performance /spectrométrie de masse)

Les analyses ont été réalisées en utilisant soit un spectromètre de masse Micromass AutoSpec OA-TOF connecté à un système HPLC Waters 2690 ou un spectromètre de masse Finnigan TSQ-700 triple quadrupole connecté à un système HPLC Waters consistant en un autosampler 757, une pompe 600-MS et un détecteur UV 486-MS. La colonne HPLC utilisée est une Nucléosil 100 5-C18 (250 x 4 mm, Macherey Nagel). Le solvant A utilisé est de l'eau contenant 0,1 % d'acide tri-fluoro-acétique (TFA), le solvant B est de l'acétonitrile contenant 0,1% TFA. Le débit est fixé à 1 ml/min. L'élution est réalisée en mode isochratique (10% A, 90% B) ou à l'aide d'un gradient linéaire de 90% A / 10% B vers 10% A / 90% B en 25 minutes suivi de 5 minutes avec 10% A / 90% B. Une post colonne de dérivation permet de diriger le mélange à un débit de 0,1 ml/min vers les spectromètres de masse. Ces derniers fonctionnent avec un électrospray fixé à 4 kV. Les spectres de masse sont acquis entre 100 et 800 Da en mode positif.

### CG/SM (Chromatographie gazeuse / Spectroscopie de masse)

Analyses réalisées à l'aide d'un chromatographe gaz HP 5890 CG combiné à un spectromètre de masse Finnigan MAT 8430. Le capillaire de silice utilisé est un J&W Sci DB-5 (30 m x 0,32 mm, 0,25 µm d'épaisseur du film). Le gaz utilisé est de l'Hélium à une pression de 150 kPa. Le programme de température est de 60°C (1 min), 30°C/min jusqu'à 270°C puis 10°C/minutes jusqu'à 320°C. L'injecteur est chauffé à 250°C. Les spectres de masse sont obtenus en mode EI à 70 eV de 20 à 800 Da. Les échantillons sont injectés avant et après trimethylsilyl derivatisation réalisée à l'aide d'un mélange de pyridine et de BSTFA (1/3, v/v) à 100°C pendant 1h.

### RMN (Résonance Magnétique Nucléaire)

Les spectres sont obtenus à l'aide d'un spectromètre Brucker DPX-360 à température ambiante. Fréquence de proton 360,12 MHz, fréquence ¹³C 90,56 MHz. Techniques appliquées pour la RMN du proton : spectroscopie une dimension, spectroscopie de corrélation homonucléaire 2D, spectroscopie nucléaire Overhauser 2 D. Pour la RMN du ¹³C : spectroscopie une dimension avec et sans découplage du proton, spectroscopie de corrélation hétéronucléaire 2D avec détection de la fréquence des ¹³C. Les molécules sont dissoutes dans CDCl₃ 99,8%.

### EXEMPLE 1

### Préparation d'un extrait à partir de Pleurotus eryngii.

5 grammes de fruit séché de *Pleurotus eryngii* sont broyé et mis à macérer dans 50 ml de méthanol à 80 % pendant 1 jour à température ambiante. Le mélange est ensuite filtré sur papier et le filtrat récolté, évaporé. L'extrait brut est repris avec de l'eau distillée (50 ml) et le pH est ajusté à 3 à l'aide d'acide chlorhydrique 2N. Cet extrait aqueux est extrait en deux fois avec de l'acétate d'éthyle, volume à volume. La phase organique est séchée avec du Na2SO4 anhydre et évaporée sous vide, à 30 °C, pour enlever le solvant. L'extrait ainsi obtenu, environ 100 mg, est repris dans 4 ml de méthanol.

### EXEMPLE 2

### Préparation d'un extrait à partir de Pleurotus eous.

La même procédure que celle décrite dans l'exemple 1 est utilisée, à la différence que l'on obtient, in fine, environ 170 mg d'extrait à partir de 4.5 g de champignon séché.

### EXEMPLE 3

### Préparation d'un extrait à partir de Ganoderma lucidum.

La même procédure que celle décrite dans l'exemple 1 est utilisée, à la différence que l'on met à macérer 6 g de champignon et que l'on obtient, in fine, environ 140 mg d'extrait.

### EXEMPLE 4

### Préparation d'un extrait à partir de Grifola frondosa.

La même procédure que celle décrite dans l'exemple 1 est utilisée, à la différence que l'on met à macérer 6,8 g de champignon et que l'on obtient, in fine, environ 180 mg d'extrait.

### EXEMPLE 5

### Préparation d'un extrait à partir de Pleurotus ostreatus.

La même procédure que celle décrite dans l'exemple 1 est utilisée, à la différence que l'on obtient, in fine, environ 120 mg d'extrait à partir de 4 g de champignon séché.

### EXEMPLE 6

### Préparation d'un extrait à partir de Agrocybe aegerita.

La même procédure que celle décrite dans l'exemple 1 est utilisée, à la différence que l'on met à macérer 6,5 g de champignon et que l'on obtient, in fine, environ 290 mg d'extrait.

### EXEMPLE 7

### Préparation d'un extrait à partir de Pholiota nameko.

La même procédure que celle décrite dans l'exemple 1 est utilisée, à la différence que l'on obtient, in fine, environ 310 mg d'extrait à partir de 4 g de champignon séché.

### EXEMPLE 8

### Préparation d'un extrait à partir de Pleurotus citrinopileatus.

La même procédure que celle décrite dans l'exemple 1 est utilisée, à la différence que l'on met à macérer 7 g de champignon et que l'on obtient, in fine, environ 160 mg d'extrait.

### EXEMPLE 9

### Préparation d'un extrait à partir de Flamulina velutipes.

La même procédure que celle décrite dans l'exemple 1 est utilisée, à la différence que l'on met à macérer 2.6 g de champignon et que l'on obtient, in fine, environ 140 mg d'extrait.

### EXEMPLE 10

### Fractionnement et caractérisation physico-chimique des composés contenus dans les extraits de champignon Ganoderma lucidum.

Ceux-ci ont été choisis car les extraits bruts obtenus présentent une forte activité hypocholestérolémiante *in vitro.*

200 g de champignon sec *Ganoderma lucidum* sont broyés et macérés dans 2 litres de méthanol 80%, à température ambiante pendant 2 jours. Le mélange est filtré sur gaze et la phase liquide évaporée sous vide, à 30°C. L'extrait méthanolique obtenu (env. 12 g) est repris avec 100 ml d'eau et le pH de cette solution est ajusté à 3 à l'aide d'acide chlorhydrique 2 N. Cet extrait aqueux est extrait 3 fois volume à volume avec de l'acétate d'éthyle. La phase organique est séchée avec du Na₂SO₄ anhydre et évaporée sous vide, à 30°C, pour éliminer le solvant. L'extrait sec obtenu (6,7 g) est repris à l'aide d'un mélange éther de pétrole et méthanol 90%. L'extrait méthanolique obtenu (6,5 g) est chromatographié sur un gel de silice ; l'élution est réalisée par paliers avec 100% CHCl₃, puis 1% MeOH dans CHCl₃, puis 10% MeOH dans CHCl₃ puis 100% MeOH. La fraction 100% CHCl₃ est rechromatographiée sur gel de silice ; l'élution est effectuée par paliers avec 100% hexane, puis 5% acétate d'éthyle dans l'hexane, puis 20% acétate d'éthyle dans l'hexane, puis 50% acétate d'éthyle dans l'hexane, puis 100% acétate d'éthyle. Les fractions actives 20% acétate d'éthyle dans l'hexane, 50% acétate d'éthyle dans l'hexane et 100% acétate d'éthyle sont purifiées par chromatographie liquide haute performance (HPLC). A cette fin, une colonne Nucleosil 100-5 C18 (250 x 4 mm, Macherey Nagel)est utilisée avec une post-colonne Lichrospher 100 RP-18 (Merck). La phase mobile consiste en un mélange de 0,05% H₃PO₄ dans eau/acétonitrile (10/90) v/v. L'élution chromatographique est réalisée en mode isocratique avec un débit de 1 ml/min. Le détecteur utilisé est un Hewlett Packard G1315A, série 1100 et λmax est fixée à 254 nm. Les molécules actives sont identifiées par Spectroscopie de Masse (SM) et Résonance Magnétique Nucléaire (RMN).

### EXEMPLE 11

### Fractionnement et caractérisation physico-chimique des composés contenus dans les extraits de champignon Pleurotus citrinopileatus.

Ceux-ci ont été choisis car les extraits bruts obtenus présentent une forte activité hypocholestérolémiante *in vitro*.

200 g de champignon sec *Pleurotus citrinopileatus* sont broyés et macérés dans 2 litres de méthanol 80%, à température ambiante pendant 2 jours. Le mélange est filtré sur gaze et la phase liquide évaporée sous vide, à 30°C. L'extrait méthanolique obtenu (env. 31 g) est repris avec 100 ml d'eau et le pH de cette solution est ajusté à 3 à l'aide d'acide chlorhydrique 2 N. Cet extrait aqueux est extrait 3 fois volume à volume avec de l'acétate d'éthyle. La phase organique est séchée avec du Na₂SO₄ anhydre et évaporée sous vide, à 30°C, pour éliminer le solvant. L'extrait acétate d'éthyle (6,6 g) est chromatographié sur un gel de silice ; l'élution est réalisée par paliers avec 100% CHCl₃, puis 5% MeOH dans CHCl₃, puis 10% MeOH dans CHCl₃ puis 100% MeOH. La fraction 100% CHCl₃ est rechromatographiée sur gel de silice ; l'élution est effectuée par paliers avec 100% hexane, puis 5% acétate d'éthyle dans l'hexane, puis 20% acétate d'éthyle dans l'hexane, puis 50% acétate d'éthyle dans l'hexane, puis 100% acétate d'éthyle. Les fractions actives 20% acétate d'éthyle dans l'hexane, 50% acétate d'éthyle dans l'hexane et 100% acétate d'éthyle sont purifiées par chromatographie liquide haute performance (HPLC). A cette fin, une colonne Nucleosil 100-5 C18 (250 x 4 mm, Macherey Nagel)est utilisée avec une post-colonne Lichrospher 100 RP-18 (Merck). La phase mobile consiste en un mélange de 0,05% H₃PO₄ dans eau/acétonitrile (10/90) v/v. Le débit est de 1 ml/min. Le détecteur utilisé est un Hewlett Packard G1315A, série 1100 et λmax est fixée à 254 nm.
Les molécules actives sont identifiées par spectroscopie de masse couplée à une chromatographie en phase gazeuse CG/SM.

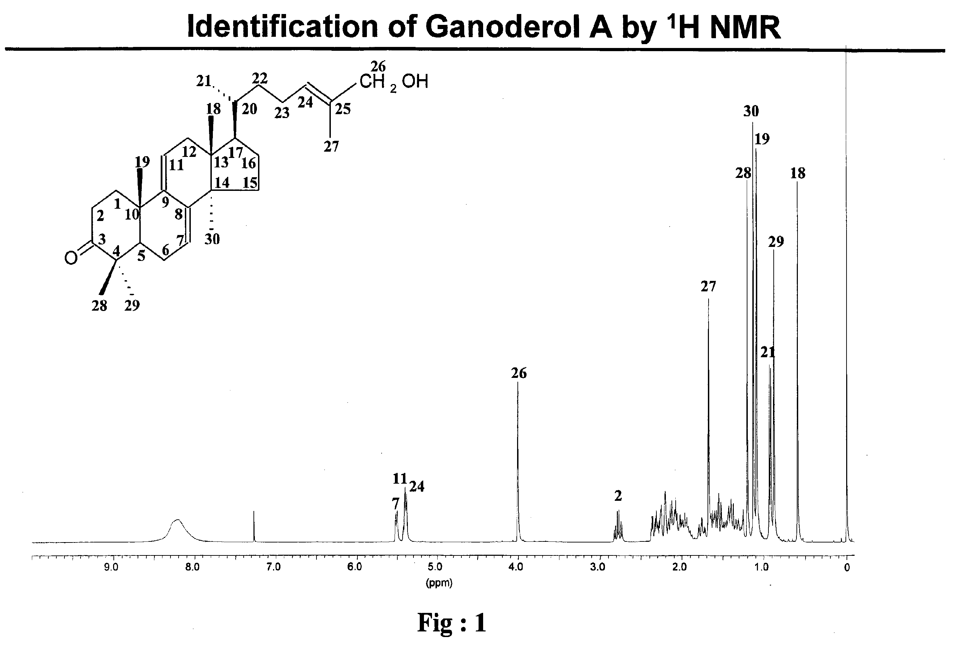

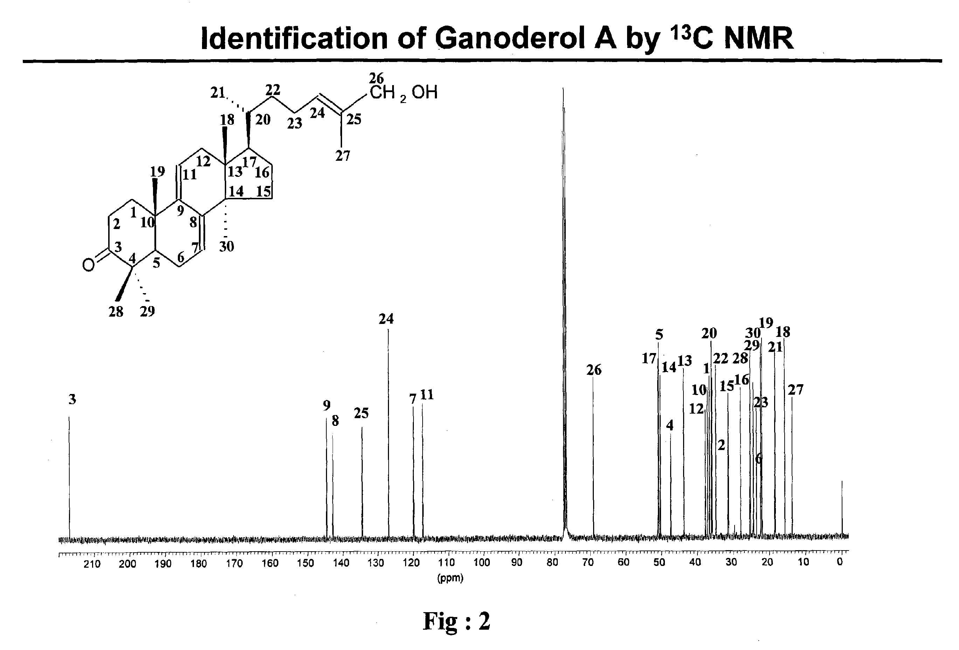

Le tableau 3 met en évidence les dérives naturels de lanostérols oxygénés (oxylanostérols) identifiés dans différentes fractions purifiées à partir de *Ganoderma lucidum* et à partir de *Pleurotus citrinopileatus.* En ce qui concerne *Ganoderma lucidum,* les analyses de spectrométrie de masse et de résonance magnétique nucléaire ont été réalisées avec les fractions 80% Hexane / 20% Acétate d'éthyle et 50% hexane / 50% acétate d'éthyle (telles qu'indiquées au tableau 2). En ce qui concerne *Pleurotus citrinopileatus,* seule la fraction 80% hexane / 20% acétate d'éthyle a été utilisée. Les figures 1 et 2 mettent en évidence le Ganoderol A identifié par RMN et spectrométrie de masse, dans les fractions purifiées de *Ganoderma lucidum* (80/20 et 50/50 Hexane/Acétate d'éthyle) et de *Pleurotus citrinopileatus* (80/20 Hexane/Acétate d'éthyle).

**Tableau 1 :**

| Activité hypocholestérolémiante *in vitro* observée sur cellules hépatiques humaines avec les extraits obtenus à partir de champignons comestibles. | |
|---|---|
| Champignons | ID₅₀ (µg/ml) |
| *Pleurotus eryngii* | >150 |
| *Pleurotus eous* | >70 |
| *Ganoderma lucidum* | 1 |
| *Grifola frondosa* | 180 |
| *Pleurotus ostreatus* | 150 |
| *Agrocybe aegerita* | >150 |
| *Pholiota nameko* | >300 |
| *Pleurotus citrinopileatus* | 10 |
| *Flamunila velutips* | <14 |

**Tableau 2 :**

| Activité hypocholestérolémiante *in vitro* observée sur cellules hépatiques humaines avec les fractions purifiées des extraits obtenus à partir des champignons comestibles *Ganoderma lucidum* et *Pleurotus citrinopileatus.* | |
|---|---|
| Fractions | ID₅₀ (µg/ml) |
| *Ganoderma lucidum* | |
| 100% CHCl₃ | |
| 95% Hexane-5% Acétate d'éthyle | >17 |
| 80%Hexane-20% Acétate d'éthyle | 3 |
| 50%Hexane-50% Acétate d'éthyle | 0.8 |
| 100% Acétate d'éthyle | 1.5 |
| 95% CHCl₃-5% MeOH | 1.3 |
| 90% CHCl₃-10% MeOH | 2.4 |
| 100% MeOH | >2 |

| *Pleurotus citrinopileatus* | |
|---|---|
| 100% CHCl₃ | |
| 95% Hexane-5% Acétate d'éthyle | >16 |
| 80% Hexane-20% Acétate d'éthyle | 7.5 |
| 50% Hexane-50% Acétate d'éthyle | 5 |
| 100% Acétate d'éthyle | 2.5 |
| 95% CHCl₃-5% MeOH | 15 |
| 90% CHCl₃-10% MeOH | >60 |
| 100% MeOH | >55 |

**Tableau 3 :**

| Activité hypocholestérolémiante *in vitro* observée sur cellules hépatiques humaines avec les principales molécules d'oxylanostérol purifiées des extraits obtenus à partir des champignons comestibles *Ganoderma lucidum* et *Pleurotus citrinopileatus.* | | |
|---|---|---|
| Oxylanosterols | Masse (m/z) | ID₅₀ (µg/ml) |
| ^{a}Ganoderol A | 438 | 1 |
| ^{b}Ganoderal A | 436 | 7 |
| ^{b}Ganoderol B | 440 | 10 |
| ^{b}acide Ganodérique Y | 454 | 0.5 |

| | | |
|---|---|---|
| ^{a} molécule présente dans *Ganoderma lucidum* et *Pleurotus citrinopileatus* | | |
| ^{b} molécule présente juste *Ganoderma lucidum.* | | |

## Revendications

1. Agent hypocholestérolémiant riche en dérivés de lanostérol naturels oxygénés isolés de champignons comestibles.

2. Agent hypocholestérolémiant selon la revendication 1, **caractérisé en que** les champignons sont choisis dans le groupe comprenant les ordres de classification : Agaricalles, Aphyllophorales et Stereales.

3. Agent hypocholestérolémiant selon les revendications 1 et 2, **caractérisé en ce que** les champignons sont choisis dans le groupe comprenant : *Pleurotus eryngii, Pleurotus eous, Ganoderma lucidum, Grifola frondosa, Pleurotus ostreatus, Agrocybe aegerita, Pholiota nameko, Pleurotus citrinopileatus et Flamulina velutipes.*

4. Utilisation dans un produit alimentaire, d'un agent hypocholestérolémiant selon les revendications 1 à 3.

5. Procédé de préparation d'un agent hypocholestérolémiant selon la revendication 1, comprenant les étapes suivantes :
- macération d'un champignon comestible dans un premier solvant,
- séparation des phases solides et liquides,
- évaporation de la phase liquide jusqu'à obtention d'un extrait sec,
- reprise de l'extrait sec avec de l'eau,
- extraction de la phase aqueuse à l'aide d'un second solvant de polarité inférieure au premier solvant et non miscible avec l'eau et
- séparation des phases aqueuse et organique.

6. Procédé selon la revendication 5, **caractérisé en ce que** le premier solvant est choisi dans le groupe comprenant le méthanol, l'éthanol et le chloroforme, utilisé seuls ou en mélange.

7. Procédé selon la revendication 5, **caractérisé en ce que** le deuxième solvant est choisi dans le groupe comprenant l'acétate d'éthyle, l'isopropanol et le chloroforme, utilisés seuls ou en mélange.

8. Procédé selon la revendication 5, **caractérisé en ce que** l'étape de macération est réalisée pendant 4 à 96 heures à une température comprise entre 5 et 30 °C.

9. Procédé selon la revendication 5, **caractérisé en ce qu'**on ajuste le pH de la phase aqueuse à une valeur comprise entre 2 et 5 avant l'extraction à l'aide du second solvant.
